# EUROPÄISCHE PATENTSCHRIFT

(11) **EP 2 272 437 B1**
(45) Veröffentlichungstag und Bekanntmachung des Hinweises auf die Patenterteilung: **10.08.2016**
(21) Anmeldenummer: 10007011.9
(22) Anmeldetag: 07.07.2010
(51) Int. Cl.: A61B 17/02

(54) **Chirurgisches Spreizinstrument**
Surgical spreading instrument
Instrument écarteur chirurgical

(30) Priorität: 11.07.2009 DE 202009009503 U
(43) Veröffentlichungstag der Anmeldung: 12.01.2011
(73) Patentinhaber: Gottfried Storz, Medizintechnik GmbH & Co. KG, 78532 Tuttlingen (DE)
(72) Erfinder: Kreidler, Winfried, 78532 Tuttlingen (DE)
(74) Vertreter: Binner, Bernhard

(56) Entgegenhaltungen:
- US-A- 1 706 500
- US-A- 5 512 038
- US-A- 5 888 197
- US-A- 5 897 087
- US-A- 6 033 363
- US-A1- 2007 073 111
- US-A1- 2008 114 209

## Beschreibung

Die Erfindung betrifft ein chirurgisches Spreizinstrument für operative Körperöffnungen, das mit zwei sich gegenüber stehenden Spreizklauen versehen ist, die gegeneinander verstellbar mittels je einem Trägerarm an einer Trägerschiene befestigt sind, wobei wenigstens eine der Spreizklauen an ihrem Trägerarm um eine Schwenkachse schwenkbar gelagert ist, die im Wesentlichen quer zur Trägerschiene und parallel zum Trägerarm verläuft und mittels einer Stelleinrichtung in Bezug auf die gegenüberliegende Spreizklaue durch Verschwenken zur Vergrößerung der Spreizstellung in unterschiedliche Winkelstellungen einstellbar ist. Derartige Spreizinstrumente, wie sie beispielsweise aus DE 74 38 149 U1 bekannt sind, werden bei chirurgischen Eingriffen zum Auseinanderziehen von Wundrändern benutzt. Dabei werden die beiden gegeneinander verstellbaren Spreizklauen (auch Valven oder Wundhaken genannt) in die zu öffnende Wunde eingeführt und danach mittels geeigneter Antriebsmittel auseinander geschoben, so dass eine spreizende Aufweitung der Wundöffnung entsteht. Bei diesem bekannten Spreizinstrument ist die Trägerschiene als Zahnstange ausgebildet, mit deren Verzahnung ein Zahnritzel in Eingriff steht, das im Führungsteil eines auf der Trägerschiene beweglich gelagerten Trägerarms gelagert und mit einem Drehgriffteil versehen ist. Der zweite Trägerarm ist dabei am einen Ende der Trägerschiene unbeweglich befestigt. Neuere Ausführungen solcher Spreizinstrumente sind zudem mit einer Sperrklinke versehen, die mit der Verzahnung der Zahnstange in Eingriff gebracht werden kann, um eine durch Drehen des Zahnritzels eingestellte Position des beweglichen Trägerarms an der Trägerschiene zu arretieren.

Bei diesem bekannten Spreizinstrument sind die Spreizklauen durch formschlüssige Führungen zwar abnehmbar, aber weder schwenkbar noch drehbar mit den sie tragenden Trägerarmen (dort "Spreizarme" genannt) verbunden. Es ist jedoch aus DE 35 09 787 A1 ein Spreizinstrument bekannt, bei dem die Spreizklauen durch zylindrische Befestigungszapfen an den sie tragenden Trägerarmen befestigt werden können. Dabei sind die Befestigungszapfen jeweils in der Mitte eines am oberen Ende der jeweiligen Spreizklaue rechtwinklig abgebogenen Plattenteils so befestigt, dass ihre Achsen bei Horizontallage der Trägerarme vertikal verlaufen. Die Befestigungszapfen sind in Passbuchsen der Trägerarme aufgenommen, so dass sich die Spreizklauen um die Achsen der Befestigungszapfen gegenüber den Trägerarmen verdrehen und ihre Lage dem Verlauf des Wundrandes anpassen können. Falls die Drehbarkeit unerwünscht ist, können die Spreizklauen durch Exzenterstifte oder Klemmschrauben gegen Drehung gesichert werden. Aus DE 35 09 787 A1 sind auch schon zweiteilige Spreizklauen (Valven) solcher Spreizinstrumente bekannt, deren effektive Länge durch eine Verstelleinrichtung verändert werden kann. Damit soll die Möglichkeit geschaffen werden, die Spreizklauen auf unterschiedliche Wundtiefen einzustellen, um tiefer liegende Operationsräume besser zugänglich zu machen.

Bei diesen meisten bekannten Spreizinstrumenten nehmen die Spreizklauen bei jedem einstellbaren Abstand die gleiche Winkellage zueinander ein. Die bewegliche Spreizklaue ist nur parallel zu sich selbst entlang der Trägerschiene verschiebbar. Deshalb ist eine Erweiterung des Operationsfeldes in der Tiefe, d.h. im Endbereich der Spreizklauen, auch dann nur begrenzt möglich, wenn, wie bekanntlich praktiziert, die unteren Abschnitte der in die Wunde einzuführenden Spreizklauen etwas auseinander gebogen und mit Zinken versehen sind, die zusätzlich nach außen gebogen sind. Weil die Wundöffnung, in welche die beiden Spreizklauen in der Stellung ihres Minimalabstandes eingeführt werden müssen, ursprünglich eng ist, dürfen die unteren Enden der Spreizklauen bzw. die Spitzen ihrer Zinken bei der Einführung in die zu spreizende Wundöffnung in Spreizrichtung einen nur geringen Abstand voneinander aufweisen.

Aus US 6224545 B1 ist eine Spreizvorrichtung der gattungsgemäßen Art bekannt, bei der die oberen Enden der beiden Spreizklauen an zwei parallelen Rahmenschenkeln eines Spreizrahmens befestigt sind. Dabei ist ein Rahmenschenkel im Gestellrahmen gegenüber dem anderen Rahmenschenkel horizontal auf verschiedene Abstände einstellbar und zudem um eine horizontale Achse, die parallel zum andern Rahmenschenkel verläuft, verschwenkbar. Beide Rahmenschenkel sind jeweils mit Hebelarmen versehen, in denen sich manuell betätigbare Stellschrauben befinden, mit denen die Rahmenschenkel und die an ihnen befestigten Spreizklauen in unterschiedliche Schwenkstellungen bringbar sind. Dabei stützen sich die Stellschrauben auf unbeweglichen Teilen des Gestellrahmens ab. Die Stellschrauben sind jeweils in Muttergewinde der Hebelarme der beiden Rahmenschenkel eingeschraubt und oberseitig mit Drehgriffen versehen.

Mit diesen Drehgriffen lassen sich die Stellschrauben zwar leicht betätigen, um die an den Rahmenschenkeln ihrer Hebelarme befestigten Spreizklauen in unterschiedliche Schwenklagen zu verstellen. An der Drehstellung der Stellschrauben bzw. ihrer Drehgriffe lässt sich jedoch die jeweilige Schwenkstellung einer Spreizklaue nicht erkennen.

Es besteht somit bei dieser Spreizvorrichtung die Gefahr, dass der Chirurg ungewollt eine Verstellung der einen oder anderen Spreizklaue in die falsche Richtung vornimmt, weil er die aktuelle Ausgangsschwenklage nicht erkennen kann.

Aus der US 5 897 087 A ist des Weiteren ein chirurgisches Spreizinstrument nach dem Oberbegriff des Anspruches 1 bekannt. Bei diesem Spreizinstrument sind zur Verbindung zwischen einer Trägerschiene und zwei Trägerarmen Klemmvorrichtungen vorgesehen. Des weiteren stehen die Spreizklauen ebenfalls über als Klemmvorrichtungen ausgebildete Stelleinrichtungen mit dem jeweils zugehörigen Tragarm in Verbindung. Zur Verstellung der Spreizstellung der Spreizklauen ist hier zunächst die zugehörige Stelleinrichtung zu lösen. Anschließend kann die nun frei bewegliche Spreizklaue zusammen mit der Stelleinrichtung in eine neue Spreizposition gebracht werden. Sodann wird die Stelleinrichtung wieder in der neu eingestellten Position fixiert. Bei diesem Spreizinstrument sind die erforderlichen Spreizstellungen der Spreizklauen schwierig einzustellen.

Der Erfindung liegt die Aufgabe zugrunde, ein Spreizinstrument der gattungsgemäßen Art, dessen Spreizklauen in der Stellung ihres kleinsten Abstandes leicht in eine enge Wundrandöffnung eingeführt und anschließend nicht nur durch Vergrößerung ihres gegenseitigen Abstandes zur Aufweitung der Wundöffnung benutzt werden können, derart zu verbessern, dass sich die Spreizklauen leicht und auf einfache Art mit Mitteln in unterschiedliche Schwenklagen bringen lassen, an deren Stellung auch die jeweilige Schwenklage der Spreizklauen erkennbar ist.

Gelöst wird diese Aufgabe erfindungsgemäß zusammen mit den Merkmalen des Oberbegriffes des Anspruches 1 dadurch, dass die Stelleinrichtung einen mittels eines Schwenkhebels verstellbaren Exzenternocken aufweist, der direkt oder über ein Übertragungselement auf eine exzentrisch zur Schwenkachse angeordnete Druckfläche der Spreizklaue derart einwirkt, dass durch den Exzenternocken beim Verschwenken des Schwenkhebels mit seinem Exzenternocken aus einer Ruhelage in eine maximale Arbeitslage gleichzeitig eine Verschwenkung der Spreizklaue in ihre maximale Schwenklage bewirkt wird.

Da es auch Spreizinstrumente der gattungsgemäßen Art gibt, bei denen die Trägerarme aus mehreren Gelenkabschnitten bestehen, deren Gelenkachsen parallel zur Trägerschiene verlaufen, ist darauf hinzuweisen, dass die Schwenkachse bzw. Schwenkachsen der Spreizklaue(n)jeweils möglichst parallel zu dem Gelenkabschnitt eines Trägerarms verlaufen sollte(n), an dem die Spreizklaue direkt befestigt ist. Dadurch lässt sich die Schwenkbarkeit der Spreizklaue(n) am zuverlässigsten handhaben.

Die Spreizklauen eines solchen Spreizinstruments lassen sich bei einer relativ kleinen bzw. engen Wundöffnung leicht in diese einführen, weil die beiden Spreizklauen bei kleinstem Abstand und mit nur geringfügig abgebogenen Endabschnitten und/oder mit den üblicherweise nach außen gebogenen Spreizzinken wenig Raum beanspruchen. Durch die schwenkbare Lagerung der Spreizklauen lassen sich diese, nachdem sie in die zu behandelnde Wunde eingeführt sind, vor oder nach dem Auseinanderschieben der Trägerarme so verschwenken, dass ihre Endabschnitte in der Tiefe der Wundöffnung einen größeren Freiraum erzeugen als er dem Abstand der beiden Trägerarme entspricht.

Von Vorteil ist dabei die geringe Raumbeanspruchung der beiden Spreizklauen in ihrer Ausgangsposition und auch bei ihrem Entfernen aus der Wundöffnung.

Wichtig für eine einfache Handhabung des Spreizinstruments ist auch eine leichte und sichere Bedienbarkeit der Stelleinrichtung, mit der die Spreizklaue(n) zur Vergrößerung der Spreizstellung verstellbar ist (bzw. sind).

Mit der in der einfachsten Ausführung angegebenen Stelleinrichtung lässt sich eine Spreizklaue bzw. lassen sich die Spreizklauen durch einfaches Umlegen bzw. Verschwenken eines Schwenkarms aus ihrer Ausgangsposition in ihre maximale Schwenkposition oder in Zwischenstellungen verschwenken und in der jeweils gewählten und an der Stellung des Schwenkhebels erkennbaren Position festlegen.

Die Größe des maximalen Schwenkwinkels lässt sich auf einfache Weise durch die entsprechende Gestaltung der Form bzw. der Exzentrizität des Exzenternockens festlegen. Auch bei der Verwendung eines Exzenternockens als Stellglied ist es möglich, durch stufenweise Formgebung des Exzenternockens die Spreizklaue auf Zwischenpositionen einzustellen. Zudem kann der Chirurg an der jeweiligen Stellung des Schwenkhebels leicht und zuverlässig erkennen, in welcher Schwenklage sich die Spreizklaue(n) befindet bzw. befinden. An einer Stellschraube lässt sich die jeweils eingestellte Schwenklage einer Spreizklaue nicht erkennen.

Dazu ist es zweckmäßig, den Schwenkhebel gemäß Anspruch 2 in der maximalen Schwenklage oder in Zwischenpositionen der Spreizklaue mit einem Rasteingriff zu sichern.

Statt oder zusätzlich zu einem Rasteingriff, der die Spreizklaue in ihrer maximalen Schwenklage sichert, kann gemäß Anspruch 3 ein Sperrriegel vorgesehen sein, der mit dem Schwenkhebel bzw. dessen Exzenternocken in Eingriff gebracht werden kann.

Es kann auch zweckmäßig sein, gemäß Anspruch 4 einen Schwenkwinkelbegrenzer vorzusehen, der eine Überdehnung des zu spreizenden Gewebes verhindert.

Von Vorteil ist in jedem Fall das Vorsehen einer Rückstellfeder gemäß Anspruch 5, durch welche nach entsprechender Betätigung des Stellgliedes eine selbsttätige Rückkehr der Spreizklaue in ihre Ausgangslage erreicht werden kann.

Die Ausgestaltung der Erfindung nach Anspruch 6 ist nicht nur hinsichtlich einer einfachen Herstellung von Vorteil, sondern es lässt sich durch sie auch auf einfache Weise die erforderliche Stabilität und Belastungsfähigkeit erzielen.

Durch die Ausgestaltung nach Anspruch 7 lassen sich die Scharnierteile leicht montieren und voneinander trennen, beispielsweise um einen Austausch einer Spreizklaue vorzunehmen.

Eine sich insbesondere durch geringe Raumbeanspruchung auszeichnende Anordnung ist Gegenstand des Anspruchs 8, die zudem den an sich bekannten Vorteil bietet, dass der Kupplungszapfen sich zusammen mit seiner Spreizklaue vom Scharnierteil lösen lässt. Eine besonders einfache Ausführungsform der Erfindung ist durch die Merkmale der Ansprüche 9 und 10 gekennzeichnet.

Anhand der Zeichnungen wird die Erfindung im Folgenden näher erläutert. Dabei zeigt:
- Fig. 1: ein chirurgisches Spreizinstrument in 3D-Ansicht;
- Fig. 2: in 3D-Ansicht eine Spreizklaue mit an einem Endabschnitt eines Trägerarms befestigten Scharnierteilen;
- Fig. 3: in vergrößerter 3D-Ansicht den mit dem Endabschnitt des Trägerarms verbundenen Scharnierteil;
- Fig. 4: in vergrößerter 3D-Ansicht den zweiten mit der Spreizklaue verbundenen Scharnierteil;
- Fig. 5: eine Scharnierwelle und zwei Scharnierzapfen;
- Fig. 6: in 3D-Ansicht eine Spreizklaue mit an einem Endabschnitt eines Trägerarms befestigten anderen Scharnierteilen;
- Fig. 7: in 3D-Ansicht die Spreizklaue aus Fig. 6 mit ihrem Scharnierteil;
- Fig. 8: teilweise im Schnitt die beiden als Paar zusammengehörigen Spreizklauen mit ihren Stelleinrichtungen in unterschiedlichen Schwenkstellungen;
- Fig. 9: einen vergrößerten Ausschnitt aus Fig. 8 ohne Schwenkarm;
- Fig. 10: den Hohlzapfen aus Fig. 9 im Schnitt als Einzelteil;
- Fig. 11: eine Stirnansicht XI aus Fig. 10;
- Fig. 13: im Schnitt die beiden Scharnierteile mit einem Schwenkwinkelbegrenzer und einer Rückstellfeder nach einer Schnittlinie VIII - VIII aus Fig. 2;
- Fig. 14: die gleichen Teile wie Fig. 13, jedoch in der maximalen Schwenkstellung des zweiten Scharnierteils;
- Fig. 15: in vergrößerter isometrischer Darstellung die Scharnierteile der Fig. 2 in der Ausführung mit einem Sperrriegel;
- Fig. 16: die Teile der Fig. 15 im Schnitt;
- Fig. 17: die Teile der Fig. 16 mit einer anderen Ausführung des Exzenternockens;
- Fig. 18: den Schwenkhebel der Fig. 17 als Einzelteil etwas vergrößert;
- Fig. 19: teilweise geschnitten zwei als Paar zusammengehörige Spreizklauen mit anderen Scharnieren und anderen Stelleinrichtungen in unterschiedlichen Schwenkstellungen;
- Fig. 21: die rechte Spreizklaue aus Fig. 19 in 3D - Darstellung;
- Fig. 22: die Einzelteile der zur rechten Spreizklaue der Fig. 19 gehörenden Stelleinrichtung sowie deren Scharnierteile.

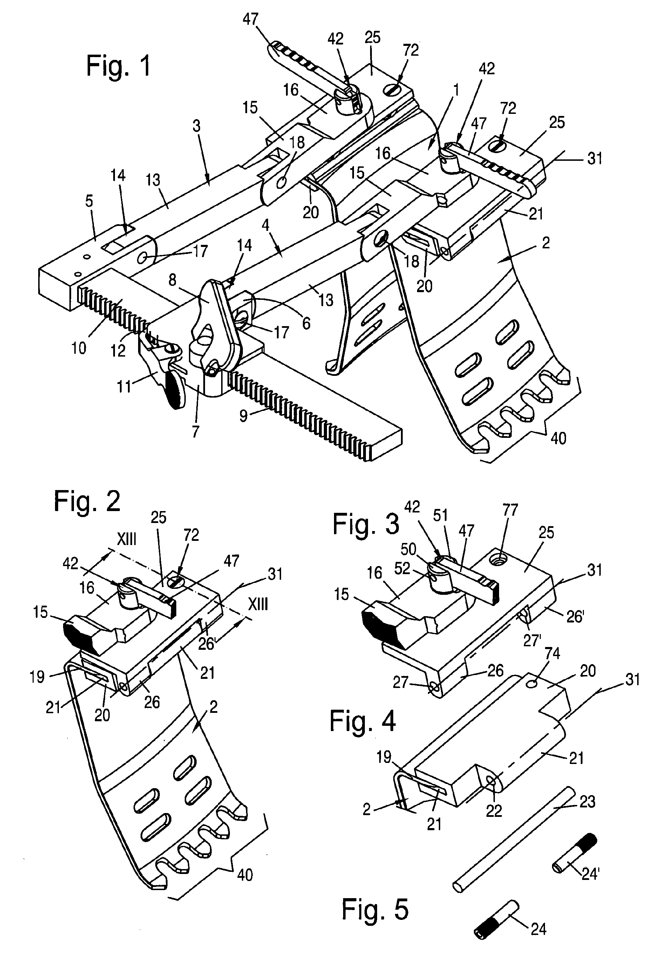

Das in Fig. 1 in 3-D-Ansicht dargestellte chirurgische Spreizinstrument für operative Körperöffnungen weist zwei sich mit ihren Rückseiten gegenüberstehende Spreizklauen 1 und 2 auf, deren obere Enden jeweils an einem Trägerarm 3 bzw. 4 befestigt sind. Der Trägerarm 3 ist mittels einer Gelenkgabel 5 festsitzend an einem Ende einer als Zahnstange ausgebildeten Trägerschiene 10 befestigt. Der Trägerarm 4 ist ebenfalls an einer Gelenkgabel 6 befestigt. Diese Gelenkgabel 6 ist jedoch mittels eines Führungsschlittens 7 verschiebbar auf der Trägerschiene 10 gelagert.

Über ein in der Zeichnung nicht dargestelltes Zahnritzel, das mit einem Drehgriff 8 versehen ist, steht der Führungsschlitten 7 mit der Verzahnung 9 der Trägerschiene 10 in Eingriff, so dass der Trägerarm 4 durch entsprechendes Drehen des Drehgriffes 8 und des Zahnritzels mit dem Führungsschlitten 7 auf der Trägerschiene 10 in beiden Richtungen verstellbar ist.

Zur Arretierung des Führungsschlittens 7 bzw. des Trägerarms 4 ist eine manuell betätigbare, gefederte Sperrklinke 11 vorgesehen, deren Sperrzunge 12 mit der Verzahnung 9 der Trägerschiene 10 arretierend in Eingriff gehalten wird. Der Trägerarm 4 mit der daran befestigten Spreizklaue 2 lässt sich somit gegenüber dem Trägerarm 3 mit der Spreizklaue 1 entlang der Trägerschiene 10 beliebig verstellen und in der jeweils gewünschten Position, d.h. in dem jeweils gewünschten Abstand von der Spreizklaue 1, arretieren.

Da die konstruktiven Elemente, durch welche die beiden Spreizklauen 1 und 2 jeweils mit einem Endstück 16 eines Trägerarms 3 bzw. 4 verbunden sind, bei allen Ausführungsbeispielen jeweils paarweise gleich sind, sind die jeweils gleichen Teile beider Trägerarme mit den gleichen Bezugszahlen versehen.

Der Trägerarm 3 weist ein Gelenkzwischenstück 13 auf, dessen eines Ende über ein Gelenk 14 mit der Gelenkgabel 5 verbunden ist und dessen anderes Ende mit einer weiteren Gelenkgabel 15 gelenkig in Verbindung steht. Diese Gelenkgabel 15 ist mit einem abgeflachten Endstück 16 versehen, an dem die Spreizklaue 1 in der nachstehend näher beschriebenen Weise befestigt ist.

In analoger Weise ist auch beim Trägerarm 4 ein Gelenkzwischenstück 13 vorgesehen, das durch ein Gelenk 14 mit der Gelenkgabel 6 des Führungsschlittens 7 verbunden ist, und dessen anderes Ende mit einer Gelenkgabel 15 in Verbindung steht. Die Gelenkgabel 15 des Trägerarms 4 ist wie die Gelenkgabel 15 des Trägerarms 3 mit einem abgeflachten Endstück 16 versehen, welches die Spreizklaue 2 trägt.

Die Gelenke 14, durch welche die Gelenkzwischenstücke 13 mit den Gelenkgabeln 5 bzw. 6 verbunden sind, sind durch Spannschrauben 17 auf einen adäquaten Reibschluss einstellbar, ebenso sind die Gelenkgabeln 15 durch Spannschrauben 18 mit den Gelenkzwischenstücken 13 einstellbar verbunden.

Wie aus den Fig. 2 bis 4 ersichtlich ist, ist das obere Ende der Spreizklaue 2 mit einer abgewinkelten Befestigungslasche 19 an einem ersten Scharnierteil 20 befestigt. Dabei ragt die Befestigungslasche 19 in eine Nut 21 des Scharnierteils 20, in der sie festsitzend beispielsweise durch Schrauben gehalten ist. Es besteht somit zwischen dem Scharnierteil 20 und der Spreizklaue 2 eine starre Verbindung.

Das Scharnierteil 20 weist einen gerundeten mittleren Gelenkvorsprung 21 mit einer Scharnierbohrung 22 auf, die eine Scharnierwelle 23 oder zwei Scharnierzapfen 24 bzw. 24' zur Bildung eines Scharniers aufnehmen kann.

An dem Endstück 16 der Gelenkgabel 15 ist ein zweites Scharnierteil 25 befestigt, das im Wesentlichen die Form einer in Gebrauchslage horizontalen Platte aufweist und unterseitig zwei Scharnieraugen 26 bzw. 26' mit koaxialen Scharnierbohrungen 27 bzw. 27' aufweist. Im zusammengesetzten Zustand bilden die beiden Scharnierteile 20 und 25 ein Scharnier, bei dem die Scharnierbohrungen 27 und 27' koaxial zur Scharnierbohrung 22 verlaufen und gemeinsam entweder die Scharnierwelle 23 oder die beiden Scharnierzapfen 24 und 24' aufnehmen.

Es ist möglich, die Scharnierwelle 23 bzw. die Scharnierzapfen 24 und 24' so auszubilden, dass sie auswechselbar montiert werden können.

Die mit Hilfe dieser beiden Scharnierteile 20 und 25 an dem Endstück 16 der Gelenkgabel 15 und somit am Trägerarm 4 befestigte Spreizklaue 2 ist auf diese Weise um eine quer zur Trägerschiene 10 und parallel zum Trägerarm 4 verlaufende Schwenkachse 31 schwenkbar gelagert.

In analoger Weise ist auch die Spreizklaue 1 über Scharnierteile 20 bzw. 25 mit dem Endstück 16 der Gelenkgabel 15 und somit mit dem Gelenkarm 3 verbunden. Auch die Spreizklaue 1 ist somit um eine im Wesentlichen quer zur Trägerschiene 10 und parallel zum Trägerarm 4 verlaufende Schwenkachse 31 schwenkbar am Trägerarm 4 gelagert.

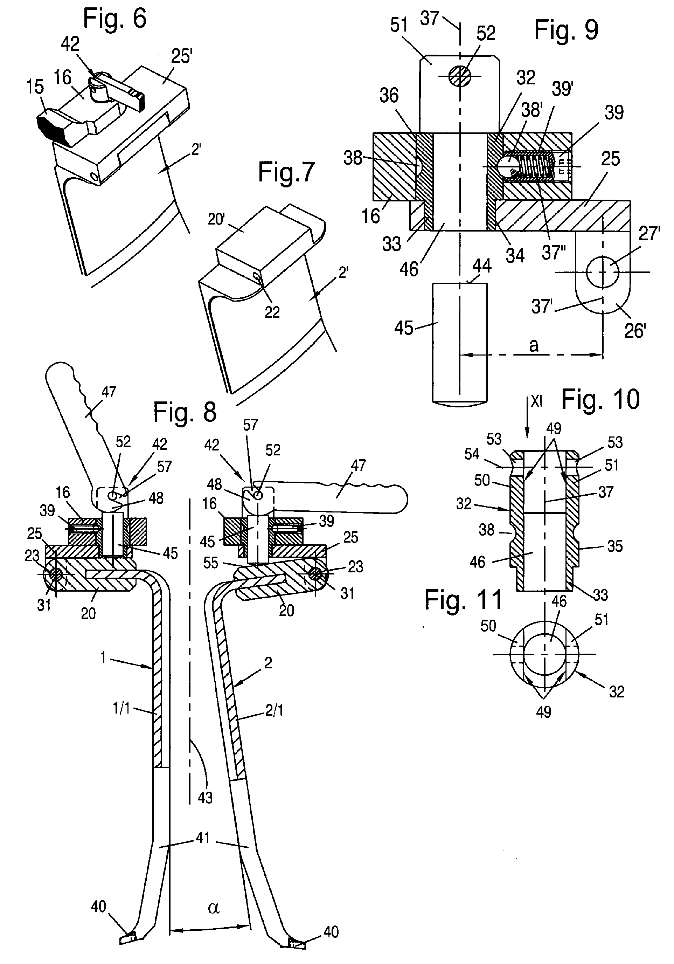

In den Fig. 6 und 7 ist eine Ausführungsform einer Spreizklaue 2' dargestellt, die an ihrem oberen Ende mit einem einstückig angeformten Scharnierteil 20' mit einer Scharnierbohrung 22 versehen ist. Dieser Scharnierteil 20' hat die Form eines Quaders, der von dem zweiten Scharnierteil 25' in der beschriebenen Weise wie das Scharnierteil 20 aufgenommen werden kann. Diese Ausführungsform bietet eine stabilere, d.h. höher belastbare, Verbindung zwischen der Spreizklaue 2' und dem Scharnierteil 25'.

Im Übrigen ist die Funktionsweise die gleiche wie bei der Ausführungsform der Fig. 1 bis 5 bzw. bei den nachstehend beschriebenen Ausführungsformen der Fig. 8.

Wie am besten aus den Fig. 8 bis 11 ersichtlich ist, besteht zwischen dem Scharnierteil 25 und dem Endstück 16 des Trägerarms 4 eine lösbare Verbindung, bei der ein Hohlzapfen 32 als Kupplungsstück dient. Der Hohlzapfen 32 ist mit einem unteren verjüngten Abschnitt 33 festsitzend in einer zylindrischen Bohrung 34 des Scharnierteils 25 befestigt und ragt mit einem zylindrischen Abschnitt 35 in eine zylindrische Bohrung 36 des Endstücks 16, so dass das Scharnierteil 25 um die Achse 37 des Hohlzapfens 32 verdrehbar ist.

Dabei ist der Hohlzapfen 32 in dem Scharnierteil 25 so angeordnet, dass seine Achse 37 von der dazu parallelen Ebene 37' der Scharnierbohrungen 27, 27' einen Abstand a aufweist, also exzentrisch zu diesen Scharnierbohrungen 27, 27' bzw. der Schwenkachse 31 angeordnet ist.

Um den Hohlzapfen 32 in axialer Richtung in der Bohrung 36 des Endstücks 16 sichern zu können, ist der Hohlzapfen 32 mit einer umlaufenden Ringnut 38 versehen, in welche eine Rastkugel 38' federnd eingreift und eine lösbare Rastverbindung zwischen dem Hohlzapfen 32 und dem Endstück 16 herstellt. Diese Rastkugel 38' ist zusammen mit einer Druckfeder 37'' in einer Hohlschraube 39 gelagert, die in einer radial zur Achse 37 verlaufenden Gewindebohrung 39' des Endstücks 16 sitzt. Somit ist der Hohlzapfen 32 mit der daran befestigten Spreizklaue 2 bzw. 2' im Endstück 16 um die Achse 37 schwenkbar und auch leicht austauschbar.

Diese Art der Befestigung des Scharnierteils 25 am Endstück 16 eines Trägerarmes 4 bzw. 3 ist bei allen Ausführungsformen des Erfindungsgegenstandes gemäß den Fig. 1 bis 17 realisiert.

Um eine oder beide Spreizklauen 1 bzw. 2 aus ihrer Normallage, in welcher die beiden geraden oberen Abschnitte 1/1 bzw. 2/1 (Fig. 8) parallel zueinander verlaufen und die Scharnierteile 20 mit ihren Oberseiten jeweils an den Unterseiten der Scharnierteile 25 anliegen, beim Gebrauch des Spreizinstrumentes um ihre Schwenkachsen 31 so verschwenken zu können, dass die unteren mit krallenartigen Zinken 40 versehenen Endabschnitte 41 Schrägstellungen mit größeren Abständen voneinander einnehmen, sind Stellvorrichtungen 42 vorgesehen. Diese im Bezug auf eine gedachte Mittelebene 43 spiegelbildlich zueinander angeordneten Stelleinrichtungen 42 (Fig. 8) bestehen jeweils aus einem Übertragungselement 45 das als Druckstößel ausgebildet ist und die Form eines zylindrischen Bolzens aufweist. Dieses Übertragungselement 45 ist axial verschiebbar in einer zylindrischen Bohrung 46 des Hohlzapfens 32 geführt und mittels eines Exzenternockens 48 betätigbar, der mit einem Schwenkhebel 47 versehen ist. Dabei ist der Exzenternocken 48 in einer Quernut 49 zwischen zwei seitlichen, parallelen Stützlaschen 50 und 51 des oberen, das Endstück 16 überragenden Teils des Hohlzapfens 32 um einen Lagerzapfen 52 schwenkbeweglich geführt. Der Lagerzapfen 52 ist in zwei koaxialen Bohrungen 53 der beiden Stützlaschen 50 und 51 befestigt (siehe auch Fig. 3). Die beiden Bohrungen 53 und der in ihnen befestigte Lagerzapfen 52 ist dabei so angeordnet, dass sich deren Achse 54 mit der Achse 37 der Bohrung 46 bzw. des Hohlzapfens 32 rechtwinklig schneidet. Zum schwenkbaren Einhängen in den Lagerzapfen 52 ist der Exzenternocken 48 mit einem offenen Schlitz 57 versehen.

Durch das Verschwenken des Schwenkhebels 47 aus der in Fig. 8 in der linken Hälfte dargestellten, nach oben gerichteten Ruhelage in die in der rechten Hälfte dargestellte horizontale Arbeitslage, wird das auf der oberen Druckfläche 55 des Scharnierteils 20 aufsitzende Übertragungselement 45 nach unten bewegt und dadurch eine Verschwenkung der Spreizklaue 1 bzw. 2 um deren Schwenkachse 31 bewirkt. Die Größe der Schwenkbewegung einer Spreizklaue 1 oder 2 kann beispielsweise auf einen Schwenkwinkel α von 10° oder weniger begrenzt sein. Durch diese Schwenkbewegung kann, wie ohne Weiteres aus Fig. 8 ersichtlich ist, der Abstand der unteren Endabschnitte 41 der beiden Spreizklauen 1 und 2 relativ stark vergrößert werden, ohne dass der Abstand der beiden Trägerarme 3 und 4 und die Öffnung des Wundrandes vergrößert wird. Der im Bereich der unteren Endabschnitte 41 liegende Operationsraum kann dadurch erheblich vergrößert und den jeweiligen Bedürfnissen angepasst werden.

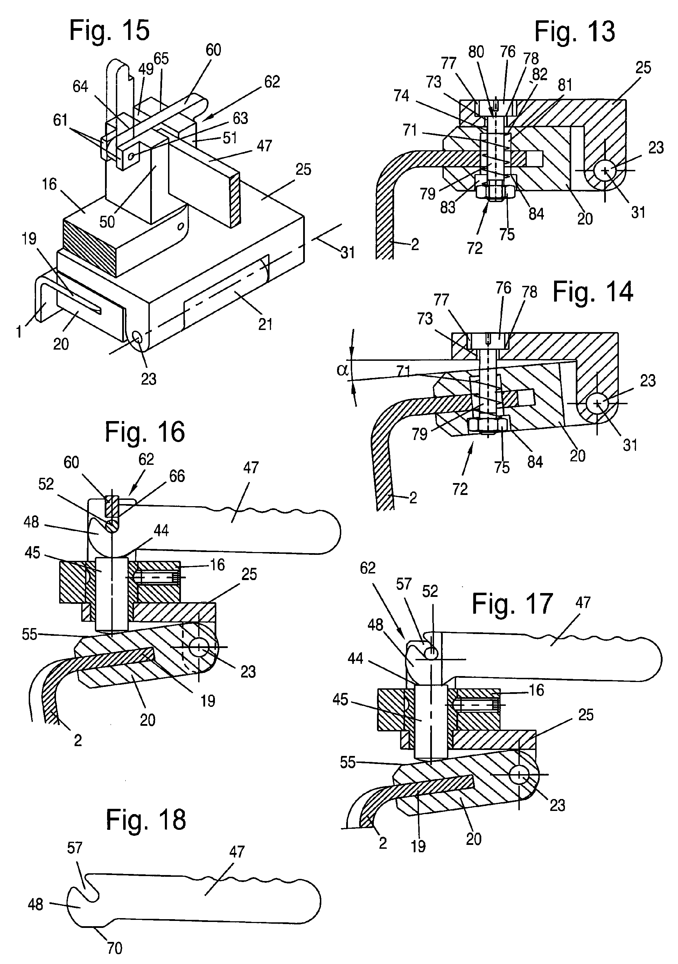

Um den Schwenkhebel 47 in der in der rechten Hälfte der Fig. 8 dargestellten Arbeitslage zu sichern, kann, wie in den Fig. 15 und 16 dargestellt ist, ein Sperrriegel 60 vorgesehen sein, der in zwei seitlichen Lagerlaschen 61 eines in diesem Falle rechteckig ausgebildeten Kopfteils 62 eines Hohlzapfens um einen horizontalen Lagerzapfen 63 schwenkbar gelagert ist. Dabei liegt der Sperrriegel 60 in seiner Sperrposition, die in Fig. 15 und Fig. 16 dargestellt ist, in horizontaler Lage in zwei oberseitig offenen Nuten 64 und 65 der beiden Stützlaschen 50 und 51, so dass sich eine Sperrfläche 66 des Schwenkhebels 47 an ihm abstützen und den Schwenkhebel 47 in seiner in Fig. 16 dargestellten Arbeitslage sichern kann.

Aus dieser in Fig. 15 und 16 dargestellten Sperrlage ist der Sperrriegel 60 in die in Fig. 15 in dünnen Linien dargestellte Vertikallage schwenkbar, so dass der Schwenkhebel 47 wieder in seine Ausgangslage zurückgeschwenkt werden kann und die Spreizklauen 1 bzw. 2 in ihre im Wesentlichen vertikale (bezogen auf Fig. 8) Ausgangslage zurückkehren können.

Eine andere Möglichkeit, den Schwenkhebel 47 in seiner Arbeitslage zu sichern, besteht darin, den Exzenternocken 48 mit einer Rastfläche 70 zu versehen, die auf der planebenen Stirnfläche 44 des als Druckstößel ausgebildeten Übertragungselementes 45 rastend aufsitzt. Allerdings ist es dazu erforderlich, dass das Übertragungselement 45 unter dem Einfluss einer Rückstellfeder steht. Eine solche Rückstellfeder 71 ist Bestandteil eines in den Fig. 13 und 14 dargestellten Schwenkwinkelbegrenzers 72. Dieser Schwenkwinkelbegrenzer 72 ist in Verlängerung des Endstücks 16 des Trägerarms 3 bzw. 4 und somit exzentrisch zur Schwenkachse 31 angeordnet.

Dieser Schwenkwinkelbegrenzer 72 besteht aus einer Schraube 80, deren Schaft 79 eine Bohrung 73 des Scharnierteils 25 sowie eine dazu koaxial verlaufende Bohrung 74 des Scharnierteils 20 durchragt und an ihrem unteren Ende mit einer ggf. gekonterten Mutter 75 versehen ist.

Während der Schraubenkopf 76 in einer erweiterten oberseitig offenen Bohrung 77 sitzt und sich auf einer Ringschulter 78 abstützt, durchragt der Schaft 79 eine sich koaxial an die Bohrung 74 anschließende, erweiterte Bohrung 81, in welcher die als Schraubenfeder ausgebildete und den Schaft 79 umschließende Rückstellfeder 71 untergebracht ist. Diese Rückstellfeder 71 stützt sich einerseits an der oberen Ringschulter der erweiterten Bohrung 81 ab und unterseitig auf der Mutter 75. Somit übt die Rückstellfeder 71 auf den Scharnierteil 20 eine Rückstellkraft aus.

Zur Begrenzung der Schwenkbewegung dient die Mutter 75 als Anschlagelement. Damit die Schraube 80 unterseitig nicht zu weit aus dem Scharnierteil 20 herausragt, ist zur Aufnahme der Mutter 75 am unteren Ende der Bohrung 81 eine Bohrungserweiterung 83 vorgesehen, an deren Ringschulter 84 die Mutter 75 anliegt, wenn die maximale Schwenkposition des Scharnierteils 20 und somit der Spreizklaue 1 bzw. 2 erreicht ist.

Wie aus Fig. 17 ersichtlich ist, sitzt in dieser maximalen Schwenkposition die Rastfläche 70 des Exzenternockens 48 rastend auf der oberen Stirnfläche 44 des Übertragungselements 45 auf, wobei durch die Rückstellfeder 71 die Rastwirkung erzielt wird.

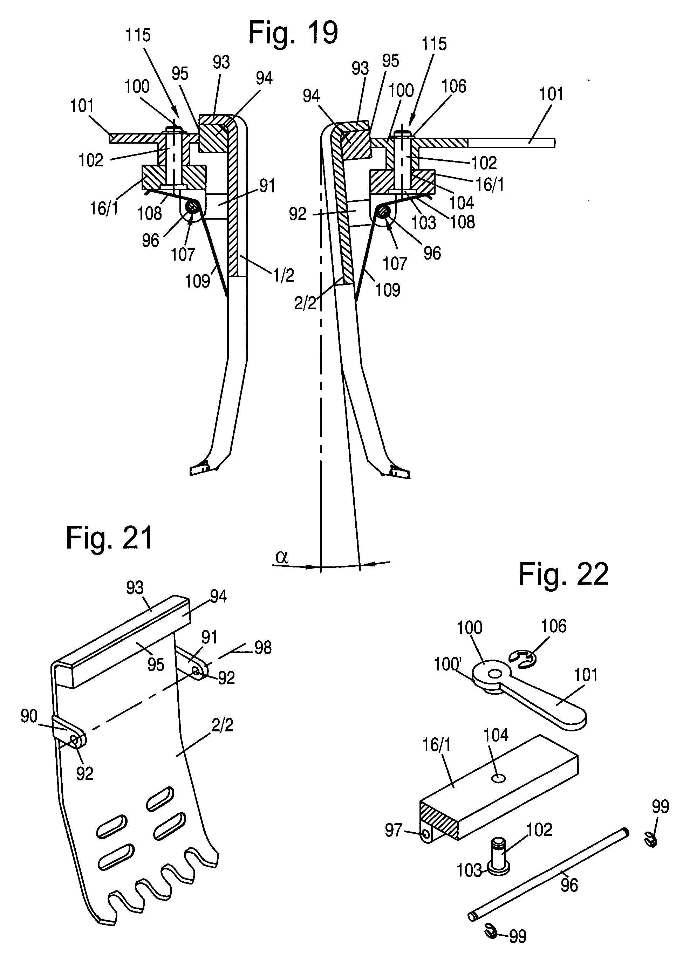

In den Fig. 19 bis 22 ist ein weiteres Ausführungsbeispiel der Erfindung mit einer anderen Stelleinrichtung 115 dargestellt. Bei dieser Ausführungsform sind die beiden Spreizklauen 1/2 und 2/2 jeweils an ihren seitlichen Rändern mit Scharnierlaschen 90 und 91 versehen, die zueinander koaxiale Bohrungen 92 aufweisen. An den oberen Enden der Spreizklauen 1/2 und 2/2 sind jeweils auf der Seite der Scharnierlaschen 90 und 91 an rechtwinklig abgebogenen Leisten 93 Druckbalken 94 angeordnet, deren außenseitige Flächen jeweils Druckflächen 95 für Betätigungselemente der Stelleinrichtung 115 bilden. Mittels einer Scharnierwelle 96, die in den Bohrungen 92 der Scharnierlaschen 90 und 91 gelagert ist und die eine Bohrung 97 eines Endstücks 16/1 eines Trägerarms 3 oder 4 durchragt, ist die Spreizklaue 1/2 bzw. 2/2 an dem Endstück 16/1 schwenkbar gelagert.

Gesichert ist die Scharnierwelle 96 durch zwei Sicherungsscheiben 99, die außerhalb der Scharnierlaschen 90 bzw. 91 in entsprechenden Nuten der Scharnierwelle 96 befestigt sind.

Zum Verschwenken der Spreizklaue 1/2 bzw. 2/2 ist bei der Stelleinrichtung 115 ein Exzenternocken 100 mit einem Schwenkhebel 101 vorgesehen, der auf einem Lagerzapfen 102 so gelagert ist, dass er an der Druckfläche 95 eines der Druckbalken 94 anliegt.

Der an seinem unteren Ende mit einem Flansch 103 versehene Lagerzapfen 102 ist in einer Bohrung 104 des Endstücks 16/1 in - bezogen auf Fig. 19 - vertikaler Lage angeordnet, so dass seine Achse rechtwinklig zur Scharnierwelle 96 und somit auch rechtwinklig zu der von den beiden Bohrungen 92 der Scharnierlaschen 90 und 91 definierten Schwenkachse 98 verläuft.

Mittels einer Sicherungsscheibe 106 ist der Lagerzapfen 102 in der in Fig. 19 dargestellten Lage fixiert.

Auf der Scharnierwelle 96 ist eine Rückstellfeder 107 gelagert, die zwei Federschenkel 108 und 109 aufweist, von denen sich der längere Federschenkel 109 an der Spreizklaue 1/2 bzw. 2/2 abstützt und der andere Federschenkel 108 unterseitig am Endstück 16/1. Dadurch wird auf die Spreizklaue 1/2 bzw. 2/2 ein Drehmoment in Rückstellrichtung ausgeübt, so dass die Druckflächen 95 jeweils an den Exzenternocken 100 federnd anliegen und während der Schwenkbewegungen des Schwenkhebels 101 der Kontur 100' des Exzenternockens 100 folgen kann.

## Patentansprüche

1. Chirurgisches Spreizinstrument für operative Körperöffnungen, das mit zwei sich gegenüber stehenden Spreizklauen (1, 2, 1/2, 2/2) versehen ist, die gegeneinander verstellbar mittels je einem Trägerarm (3, 4) an einer Trägerschiene (10) befestigt sind, wobei wenigstens eine der Spreizklauen (1, 2, 1/2, 2/2) an ihrem Trägerarm (3, 4) um eine Schwenkachse (31, 98) schwenkbar gelagert ist, die im Wesentlichen quer zur Trägerschiene (10) und parallel zum Trägerarm (3, 4) verläuft und mittels einer Stelleinrichtung (42, 115) in Bezug auf die gegenüberliegende Spreizklaue (1, 2, 1/2, 2/2) durch Verschwenken zur Vergrößerung der Spreizstellung in unterschiedliche Winkelstellungen einstellbar ist, wobei die Stelleinrichtung (42, 115) einen mittels eines Schwenkhebels (47, 101) verstellbaren Exzenternocken (48, 100) aufweist,
**dadurch gekennzeichnet,**
**dass** der Exzenternocken direkt oder über ein Übertragungselement (45) auf eine exzentrisch zur Schwenkachse (31, 98) angeordnete Druckfläche (55, 95) der Spreizklaue (1, 2, 1/2, 2/2) derart einwirkt, dass durch Verschwenken des Schwenkhebels (47, 101) eine Verschwenkung der Spreizklaue (1, 2, 1/2, 2/2) aus ihrer Ausgangsposition in ihre maximale Schwenkposition bewirkt wird.

2. Chirurgisches Spreizinstrument nach Anspruch 1, **dadurch gekennzeichnet, dass** der Schwenkhebel (47) und/oder der Exzenternocken (48) in seiner der maximalen Schwenklage der Spreizklaue (1, 2) entsprechenden Arbeitslage durch einen lösbaren Rasteingriff (44, 70) arretierbar ist.

3. Chirurgisches Spreizinstrument nach Anspruch 1 oder 2, **dadurch gekennzeichnet, dass** der Schwenkhebel (47) in seiner, der maximalen Schwenklage der Spreizklaue (1, 2) entsprechenden Arbeitslage durch einen Sperrriegel (60) arretierbar ist.

4. Chirurgisches Spreizinstrument nach einem der Ansprüche 1 bis 3, **dadurch gekennzeichnet, dass** die Stelleinrichtung (42) mit einem Schwenkwinkelbegrenzer (72) versehen ist.

5. Chirurgisches Spreizinstrument nach einem der Ansprüche 1 bis 4, **dadurch gekennzeichnet, dass** die Stelleinrichtung (42, 115) mit einer Rückstellfeder (71, 107) versehen ist.

6. Chirurgisches Spreizinstrument nach einem der Ansprüche 1 bis 5, **dadurch gekennzeichnet, dass** das obere Ende der Spreizklaue (1, 2) mit einem ersten Scharnierteil (20) versehen ist, das mittels Scharnierzapfen (24, 24') oder einer Scharnierwelle (23) schwenkbar an einem zweiten Scharnierteil (25) gelagert ist, das seinerseits an einem Endabschnitt (16) eines Trägerarms (3, 4) befestigt ist.

7. Chirurgisches Spreizinstrument nach Anspruch 6, **dadurch gekennzeichnet, dass** die Scharnierzapfen (24, 24') bzw. die Scharnierwelle (23) lösbar an einem der Scharnierteile (20, 25) befestigt sind.

8. Chirurgisches Spreizinstrument nach Anspruch 6, **dadurch gekennzeichnet, dass** ein das zweite Scharnierteil (25) mit dem Trägerarm (3, 4) verbindender Kupplungszapfen (32) vorgesehen ist, welcher als Hohlzapfen (32) ausgebildet ist, in dem ein als Druckstößel ausgebildetes Übertragungselement (45) gelagert ist.

9. Chirurgisches Spreizinstrument nach Anspruch 1, **dadurch gekennzeichnet, dass** die Spreizklaue (1/2, 2/2) an ihren seitlichen Rändern mit Scharnierlaschen (90, 91) versehen ist, welche zueinander koaxiale, eine Schwenkachse (98) der Spreizklaue (1/2, 2/2) definierende Bohrungen (92) aufweisen und
dass der Trägerarm (3, 4) ein Endstück (16/1) aufweist, welches mit einer Bohrung (97) versehen ist und
dass eine Scharnierwelle (96) vorgesehen ist, welche die Bohrung (97) des Endstückes (16/1) durchragt und über welche die Spreizklaue (1/2, 2/2) über ihre Scharnierlaschen (90, 91) am Endstück (16/1) gelagert ist.

10. Chirurgisches Spreizinstrument nach Anspruch 9, **dadurch gekennzeichnet, dass** die Spreizklaue (1/2, 2/2) an ihrem oberen Ende mit einer zur Schwenkachse (98) exzentrischen Druckfläche (95) versehen ist, die am Exzenternocken (100) anliegt.

## Claims

1. Surgical retractor for operating in openings in the body, with two mutually opposite retraction claws (1, 2, 1/2, 2/2) adjustable relative to each other and each secured on a carrier rail (10) by means of respective carrier arms (3, 4), wherein at least one of the retraction claws (1, 2, 1/2, 2/2) is mounted on its carrier arm (3, 4) so as to be pivotable about a pivot axis (31, 98), running substantially transverse to the carrier rail (10) and parallel to the carrier arm (3, 4), and, by means of an adjustment mechanism (42, 115), is adjustable by pivoting to different angle settings with respect to the opposite retraction claw (1, 2, 1/2, 2/2) in order to enlarge the retracted position, wherein the adjustment mechanism (42, 115) has an eccentric cam (48, 100) adjustable by means of a pivot lever (47, 101), **characterized in that** the eccentric cam acts directly, or via a transmission element (45), on a pressure surface (55, 95) of the retraction claw (1, 2, 1/2, 2/2) arranged eccentrically with respect to the pivot axis (31, 98), in such a way that a pivoting of the pivot lever (47, 101) causes a pivoting of the retraction claw (1, 2, 1/2, 2/2) from its initial position to its maximum pivot position.

2. Surgical retractor according to Claim 1, **characterized in that** the pivot lever (47) and/or the eccentric cam (48), in its working position corresponding to the maximum pivot position of the retraction claw (1, 2), can be locked by a releasable latching engagement (44, 70).

3. Surgical retractor according to Claim 1 or 2, **characterized in that** the pivot lever (47), in its working position corresponding to the maximum pivot position of the retraction claw (1, 2), can be locked by a safety bolt (60).

4. Surgical retractor according to one of Claims 1 to 3, **characterized in that** the adjustment mechanism (42) is provided with a pivot angle limiter (72).

5. Surgical retractor according to one of Claims 1 to 4, **characterized in that** the adjustment mechanism (42, 115) is provided with a restoring spring (71, 107).

6. Surgical retractor according to one of Claims 1 to 5, **characterized in that** the upper end of the retraction claw (1, 2) is provided with a first hinge part (20) which, by means of hinge pins (24, 24') or a hinge shaft (23), is mounted pivotably on a second hinge part (25), which is in turn secured on an end portion (16) of a carrier arm (3, 4).

7. Surgical retractor according to Claim 6, **characterized in that** the hinge pins (24, 24') or the hinge shaft (23) are secured releasably on one of the hinge parts (20, 25).

8. Surgical retractor according to Claim 6, **characterized in that** a coupling pin (32) is provided which connects the second hinge part (25) to the carrier arm (3, 4) and which is designed as a hollow pin (32), in which a transmission element (45) designed as a pressure tappet is mounted.

9. Surgical retractor according to Claim 1, **characterized in that** the retraction claw (1/2, 2/2) is provided, on its lateral edges, with hinge brackets (90, 91) which have mutually coaxial bores (92) defining a pivot axis (98) of the retraction claw (1/2, 2/2), and
**in that** the carrier arm (3, 4) has an endpiece (16/1) which is provided with a bore (97), and
**in that** a hinge shaft (96) is provided which passes through the bore (97) of the endpiece (16/1) and via which the retraction claw (1/2, 2/2) is mounted via its hinge brackets (90, 91) on the endpiece (16/1).

10. Surgical retractor according to Claim 9, **characterized in that** the retraction claw (1/2, 2/2) is provided, at its upper end, with a pressure surface (95) eccentric with respect to the pivot axis (98), which pressure surface (95) bears on the eccentric cam (100).

## Revendications

1. Instrument écarteur chirurgical pour des ouvertures de corps opératoires, qui est doté de deux griffes d'écartement (1, 2, 1/2, 2/2) placées l'une en face de l'autre, qui sont fixées à un rail porteur (10) chacune au moyen d'un bras porteur (3, 4) d'une façon réglable l'une par rapport à l'autre, dans lequel au moins une des griffes d'écartement (1, 2, 1/2, 2/2) est montée sur son bras porteur (3, 4) d'une façon pivotante autour d'un axe de pivotement (31, 98), qui est essentiellement transversal au rail porteur (10) et parallèle au bras porteur (3, 4), et est réglable dans différentes positions angulaires par pivotement, en vue de l'agrandissement de la position d'écartement, au moyen d'un dispositif de réglage (42, 115) par rapport à la griffe d'écartement opposée (1, 2, 1/2, 2/2), dans lequel le dispositif de réglage (42, 115) présente une came excentrique (48, 100) réglable au moyen d'un levier pivotant (47, 101), **caractérisé en ce que** la came excentrique agit directement ou par l'intermédiaire d'un élément de transmission (45) sur une face de pression (55, 95) de la griffe d'écartement (1, 2, 1/2, 2/2) disposée de façon excentrique par rapport à l'axe de pivotement (31, 98), de telle manière qu'un pivotement de la griffe d'écartement (1, 2, 1/2, 2/2) de sa position initiale à sa position de pivotement maximal soit provoqué par pivotement du levier pivotant (47, 101).

2. Instrument écarteur chirurgical selon la revendication 1, **caractérisé en ce que** le levier pivotant (47) et/ou la came excentrique (48) peut être bloqué(e), par un engagement d'encliquetage amovible (44, 70), dans sa position de travail correspondant à la position de pivotement maximal de la griffe d'écartement (1, 2).

3. Instrument écarteur chirurgical selon la revendication 1 ou 2, **caractérisé en ce que** le levier pivotant (47) peut être bloqué, par un verrou de blocage (60), dans sa position de travail correspondant à la position de pivotement maximal de la griffe d'écartement (1, 2).

4. Instrument écarteur chirurgical selon l'une quelconque des revendications 1 à 3, **caractérisé en ce que** le dispositif de réglage (42) est doté d'un limiteur d'angle de pivotement (72).

5. Instrument écarteur chirurgical selon l'une quelconque des revendications 1 à 4, **caractérisé en ce que** le dispositif de réglage (42, 115) est doté d'un ressort de rappel (71, 107).

6. Instrument écarteur chirurgical selon l'une quelconque des revendications 1 à 5, **caractérisé en ce que** l'extrémité supérieure de la griffe d'écartement (1, 2) est dotée d'une première partie de charnière (20), qui est montée de façon pivotante, au moyen de fiches de charnière (24, 24') ou d'une broche de charnière (23), sur une deuxième partie de charnière (25) qui est à son tour fixée à une partie d'extrémité (16) d'un bras porteur (3, 4).

7. Instrument écarteur chirurgical selon la revendication 6, **caractérisé en ce que** les fiches de charnière (24, 24') ou la broche de charnière (23) sont fixées de façon amovible à une des parties de charnière (20, 25).

8. Instrument écarteur chirurgical selon la revendication 6, **caractérisé en ce qu'**il est prévu une deuxième fiche de couplage (32) reliant la deuxième partie de charnière (25) au bras porteur (3, 4), lequel est réalisé en forme de fiche creuse (32), dans laquelle est monté un élément de transmission (45) réalisé en forme de pivot pressé.

9. Instrument écarteur chirurgical selon la revendication 1, **caractérisé en ce que** la griffe d'écartement (1/2, 2/2) est dotée sur ses bords latéraux de pattes de charnière (90, 91), qui présentent des alésages (92) coaxiaux l'un à l'autre et définissant un axe de pivotement (98) de la griffe d'écartement (1/2, 2/2), et
**en ce que** le bras porteur (3, 4) présente une pièce d'extrémité (16/1), qui est dotée d'un alésage (97), et **en ce qu'**il est prévu une broche de charnière (96), qui traverse l'alésage (97) de la pièce d'extrémité (16/1) et par laquelle la griffe d'écartement (1/2, 2/2) est montée sur la pièce d'extrémité (16/1) par l'intermédiaire de ses pattes de charnière (90, 91).

10. Instrument écarteur chirurgical selon la revendication 9, **caractérisé en ce que** la griffe d'écartement (1/2, 2/2) est dotée à son extrémité supérieure d'une face de pression excentrique (95) par rapport à l'axe de pivotement (98), qui s'applique sur la came excentrique (100).
